# EUROPÄISCHE PATENTSCHRIFT

(11) **EP 1 973 863 B1**
(45) Veröffentlichungstag und Bekanntmachung des Hinweises auf die Patenterteilung: **29.06.2016**
(21) Anmeldenummer: 06841430.9
(22) Anmeldetag: 18.12.2006
(51) Int. Cl.: C06C 7/00, C06B 41/00

(54) **ANZÜNDSATZ**
PRIMER COMPOSITION
COMPOSITION D'ALLUMAGE

(30) Priorität: 20.12.2005 DE 102005061323
(43) Veröffentlichungstag der Anmeldung: 01.10.2008
(73) Patentinhaber: RUAG Ammotec GmbH, 90765 Fürth (DE)
(72) Erfinder: BLEY, Ulrich, 90766 Fürth (DE); HAGEL, Rainer, 91058 Erlangen (DE); HOSCHENKO, Aleksej, 90765 Fürth (DE); LECHNER, Peter Simon, 90522 Oberasbach (DE)
(74) Vertreter: Uppena, Franz
(86) Internationale Anmeldenummer: PCT/EP2006/069849
(87) Internationale Veröffentlichungsnummer: WO 2007/071650

(56) Entgegenhaltungen:
- DE-A1- 19 912 622
- DE-C- 373 633
- GB-A- 1 605 333
- US-A- 3 238 076
- US-A- 3 522 320
- US-A- 6 165 294
- T. URBANSKI: "Chemistry and Technology of Explosives, Volume 1" 1985, PERGAMON PRESS , OXFORD, GREAT BRITAN , XP002447578 Seite 525 - Seite 528 Seite 539
- T. URBANSKI: "Chemistry and Technology of Explosives, Volume 3" 1985, PERGAMON PRESS , OXFORD, GREAT BRITAN , XP002447579 Seite 220
- LOKRE V L ET AL: "ELECTROSTATIC CHARGE MEASUREMENTS ON INITIATORS AND EXPLOSIVE POWDERS" PROPELLANTS EXPLOS PYROTECH OCT 1983, Bd. 8, Nr. 5, Oktober 1983 (1983-10), Seiten 146-148, XP002447505

## Beschreibung

Gegenstand der Erfindung ist ein Anzündsatz, ein Verfahren zu dessen Herstellung und die Verwendung des Anzündsatzes.

Herkömmliche Anzündsätze, die beispielsweise in Fahrzeugsicherheitssystemen eingesetzt werden, haben den Nachteil, dass sie wegen niedriger Zersetzungstemperaturen ihrer Anzündstoffe nicht im Motorraum von Kfz eingesetzt werden können. DE 19912622 offenbart einen Anzündsatz, der Kalliumstyphnat enthält. Im Motorraum eines Kfz werden Temperaturen von 140°C und mehr erreicht, was eine Zersetzungstemperatur eines Anzündstoffes von über 300°C erfordert. Kaliumdinitrobenzofuroxanat beispielsweise hat eine Zersetzungstemperatur von ca. 220°C und ist daher als Anzündstoff für diesen Zweck ungeeignet.

Aufgabe der vorliegenden Erfindung war es, einen Anzündsatz bereitzustellen, der die Nachteile des Standes der Technik überwindet. Dieser Anzündsatz soll einen Anzündstoff enthalten, der eine Zersetzungstemperatur oberhalb von 300 C hat. Weitere Aufgaben der Erfindung bestanden darin, einen Anzündsatz bereitzustellen, dessen Anzündstoff schwermetallfrei ist, dessen Anzündstoff sich sowohl für mechanische als auch für elektrische Anzündsysteme eignet, der beispielsweise in Fahrzeugsicherheitssystemen, Munition und Treibkartuschen für Bolzensetzgeräte eingesetzt werden kann und/oder dessen Herstellung durch einfache technische Verfahren möglich ist.

Erfindungsgemäß werden diese Aufgaben überraschenderweise durch die Merkmale der Ansprüche 1 und 12 gelöst. Vorzugsweise Ausgestaltungen finden sich in den Unteransprüchen.

Es wurde gefunden, dass diese Aufgaben durch einen Anzündsatz, der ein oder mehrere Alkali- und/oder Erdalkalisalze der Styphninsäure (2,4,6-Trinitro-1,3-Dihydroxybenzol) als Anzündstoff enthält, das Verfahren zur Herstellung dieses Anzündsatzes und dessen Verwendung gelöst werden. Diese Salze - nachfolgend kurz Styphnate genannt - können als Anzündstoff im Anzündsatz sowohl einzeln als auch in Mischung untereinander und/oder ggf. in Mischung mit den für Anzündsätze üblichen Zusatzstoffen wie beispielsweise Oxidationsmittel, Reduktionsmittel, Sensibilisatoren, Bindemittel, energiereiche Zuschläge, Abbrandmoderatoren und/oder Verarbeitungshilfen eingesetzt werden.

Erfindungsgemäß ist Kalium-Calcium-Styphnat. Erfindungsgemäß bevorzugt sind basisches Calciumstyphnat sowie basisches Kalium-Calcium-Styphnat.

Als Zusatzstoffe können erfindungsgemäß verwendet werden:
1. Oxidationsmittel (einzeln oder in Mischungen):
   Nitrate der Alkali- oder Erdalkalimetalle oder des Ammoniums wie Natriumnitrat oder Kaliumnitrat, Perchlorate der Alkali- oder Erdalkalimetalle oder des Ammoniums, Peroxide der Erdalkalimetalle oder des Zinks, bevorzugt Zinkperoxid.
2. Reduktionsmittel (einzeln oder in Mischungen):
   Aluminium, Titan, Titanhydrid, Bor, Borhydrid, Zirkon, Zirkonhydrid, Silicium, Graphit, Aktivkohle, Ruß, bevorzugt Titan.
3. Sensibilisatoren (einzeln oder in Mischungen):
   Tetrazen, Kaliumdinitrobenzofuroxanat, Diazodinitrophenol.
4. Bindemittel (einzeln oder in Mischungen):
   Adhesin, Cellulose sowie deren Derivate, Polyvinylbutyrale, Polynitropolyphenylen, Polynitrophenylether, Plexigum, Polyvinylacetat und Copolymere, bevorzugt Adhesin.
6. Energiereiche Zuschläge (einzeln oder in Mischungen):
   Hexogen, Oktogen, Nitropenta und Nitrocellulose.
7. Abbrandmoderatoren und Verarbeitungshilfen (einzeln oder in Mischungen):
   Nitrocellulose-Kugelpulver, Acetonylacetate, Salicylate, Silikate, Kieselgele, Bornitrid, bevorzugt Nitrocellulose-Kugelpulver.

Der erfindungsgemäße Anzündsatz zeichnet sich durch Schwermetallfreiheit, hohe thermische Stabilität und bei Verwendung von Kalium-Calcium-Styphnat als Anzündstoff durch den Gehalt an Calcium aus, welches durch Bildung von Calciumcarbonat in den Verbrennungsrückständen aufgrund der günstigen tribologischen Eigenschaften des Calciumcarbonats für Waffensysteme vorteilhaft ist. Die Zersetzungstemperatur des erfindungsgemäßen Anzündsatzes liegt oberhalb 300°C. Der erfindungsgemäße Anzündsatz lässt sich sowohl mechanisch als auch elektrisch anzünden.

Die Herstellung und Verarbeitung des erfindungsgemäßen Anzündsatzes findet nach an sich bekannten und üblichen Verfahren statt. Die einzelnen Bestandteile des Anzündsatzes werden dazu in geeigneter Weise in den erforderlichen Mengen miteinander vermischt.

Gegenstand der Erfindung ist im Einzelnen:
- ein Anzündsatz, der Kalium-Calcium-Styphnat, bevorzugt basisches Calciumstyphnat und/oder basisches Kalium-Calcium-Styphnat enthält;
- ein Anzündsatz, der neber Kalium-Calcium-Styphnat, basisches Calciumstyphnat und/oder basisches Kalium-Calcium-Styphnat ein oder mehrere Zusatzstoffe enthält, ausgewählt aus: Oxidationsmitteln, Reduktionsmitteln, Sensibilisatoren, Bindemitteln, energiereichen Zuschlägen, Abbrandmoderatoren und/oder Verarbeitungshilfen oder Mischungen aus mindestens zwei dieser Zusatzstoffe;
- ein Anzündsatz, der neben Kalium-Calcium-Styphnat, basisches Calciumstyphnat und/oder basisches Kalium-Calcium-Styphnat ein oder mehrere Zusatzstoffe enthält, wobei es sich beim Oxidationsmittel um ein oder mehrere der folgenden Stoffe handelt: Nitrate der Alkali- oder Erdalkalimetalle oder des Ammoniums wie Natriumnitrat oder Kaliumnitrat, Perchlorate der Alkali- oder Erdalkalimetalle oder des Ammoniums, Peroxide der Erdalkalimetalle oder des Zinks, bevorzugt um Zinkperoxid;
- ein Anzündsatz, der neben Kalium-Calcium-Styphnat, basisches Calciumstyphnat und/oder basisches Kalium-Calcium-Styphnat ein oder mehrere Zusatzstoffe enthält, wobei es sich beim Reduktionsmittel um ein oder mehrere der folgenden Stoffe handelt: Aluminium, Titan, Titanhydrid, Bor, Borhydrid, Zirkon, Zirkonhydrid, Silicium, Graphit, Aktivkohle, Ruß, bevorzugt um Titan;
- ein Anzündsatz, der neben Kalium-Calcium-Styphnat, basisches Calciumstyphnat und/oder basisches Kalium-Calcium-Styphnat ein oder mehrere Zusatzstoffe enthält, wobei es sich beim Sensibilisator um ein oder mehrere der folgenden Stoffe handelt: Tetrazen, Kaliumdinitrobenzofuroxanat, Diazodinitrophenol, bevorzugt um Tetrazen;
- ein Anzündsatz, der neben Kalium-Calcium-Styphnat, basisches Calciumstyphnat und/oder basisches Kalium-Calcium-Styphnat ein oder mehrere Zusatzstoffe enthält, wobei es sich beim Bindemittel um ein oder mehrere der folgenden Stoffe handelt: Adhesin, Cellulose sowie deren Derivate, Polyvinylbutyrale, Polynitropolyphenylen, Polynitrophenylether, Plexigum, Polyvinylacetat und Copolymere, bevorzugt um Adhesin;
- Anzündsatz, der neben Kalium-Calcium-Styphnat, basisches Calcium styphnat und/oder basisches Kalium-Calcium-Styphnat Er ein oder mehrere Zusatzstoffe enthält, wobei es sich bei den energiereichen Zuschlägen um ein oder mehrere der folgenden Stoffe handelt: Hexogen, Oktogen, Nitropenta und Nitrocellulose;
- ein Anzündsatz, der neben Kalium-Calcium-Styphnat, basisches Calcium styphnat und/oder basisches Kalium-Calcium-Styphnat ein oder mehrere Zusatzstoffe enthält, wobei es sich bei den Abbrandmoderatoren und Verarbeitungshilfen um ein oder mehrere der folgenden Stoffe handelt: Nitrocellulose-Kugelpulver, Acetonylacetate, Salicylate, Silikate, Kieselgele, Bornitrid, bevorzugt um Nitrocellulose-Kugelpulver;
- ein Anzündsatz, der 10 bis 90, bevorzugt 20 bis 80, besonders bevorzugt 30 bis 70 Gew.-% Kalium-Calcium-Styphnat, basisches Calciumstyphnat und/oder basisches Kalium-Calcium-Styphnat enthält;
- ein Anzündsatz, der 70 bis 99,99, bevorzugt 90 bis 99,9, besonders bevorzugt 90 bis 99 Gew.-% Kalium-Calcium-Styphnat, basisches Calciumstyphnat und/oder basisches Kalium-Calcium-Styphnat enthält;
- ein Anzündsatz, der 90 bis 99,99, bevorzugt 95 bis 99 Gew.-% Kalium-Calcium-Styphnat, vorzugsweise basisches Kalium-Calcium-Styphnat, und 0,01 bis 10, bevorzugt 1 bis 5 Gew.-% Bindemittel, vorzugsweise Adhesin, enthält;
- die Verwendung eines Anzündsatzes, der Kalium-Calcium-Styphnat, basisches Calciumstyphnat und/oder basisches Kalium-Calcium-Styphnat enthält, in Anzündsystemen für Fahrzeugsicherheitssystemen, Munition und/oder Treibkartuschen für Bolzensetzgeräte;
- die Verwendung eines Anzündsatzes, der Kalium-Calcium-Styphnat, basisches Calciumstyphnat und/oder basisches Kalium-Calcium-Styphnat enthält, in Anzündsystemen für Fahrzeugsicherheitssystemen, bevorzugt für solche, die im Motorraum eines Kfz eingesetzt werden;
- die Verwendung eines Anzündsatzes in Anzündsystemen, die elektrisch gezündet werden;

Die nachfolgenden Beispiele sollen die Erfindung näher erläutern, ohne sie einzuschränken.

### Beispiel 1: Kalium-Calcium-Styphnat:

Basisches Kalium-Calcium-Styphnat, hergestellt durch die Fällung aus Kaliumstyphnatlösung unter stöchiometrischer Zugabe von Calciumnitrat, wurde mit Atomabsorptionsspektroskopie untersucht. Es wurden folgende Anteile von Kalium und

**Calcium gefunden:**

| | |
|---|---|
| Kalium: | ca. 13 Gew.-% |
| Calcium: | ca. 7 Gew.-% |

### Beispiel 2: basisches Kalium-Calcium-Styphnat:

Kalium-Calcium-Styphnat, hergestellt durch die Zugabe von Alkalihydroxiden zu einer Kalium-Calcium-Styphnat-Suspension, wurde mit Atomabsorptionsspektroskopie untersucht. Es wurden folgende Anteile von Kalium und Calcium gefunden:

| | |
|---|---|
| Kalium: | ca. 11,5 Gew.-%. |
| Calcium: | ca. 12 Gew.-% |

In Tabelle 1 sind die Zersetzungstemperaturen, Reib- und Schlagempfindlichkeiten der Substanzen dargestellt. Die Messung der Reib- und Schlagempfindlichkeiten erfolgte nach Methoden der Bundesanstalt für Materialforschung (BAM), während die Messung der Zersetzungstemperaturen mit der Thermogravimetrie-Analyse (Fa. Mettler) bei einer Aufheizrate von 10°C pro Minute erfolgte.

**Tabelle 1:**

| | Kalium-Calcium-Styphnat | basisches Kalium-Calcium-Styphnat | basisches Calcium-styphnat |
|---|---|---|---|
| Reibempfindlichkeit in N | 9 | 9 | 9 |
| Schlagempfindlichkeit in J | 3 | 3 | 4 |
| Zersetzungstemperatur in °C | 345 | 340 | 335 |

Diese Styphnate werden mit den für Anzündsätze üblichen Zusatzstoffen nach an sich bekannten Verfahren in den erforderlichen Mengenanteilen vermischt.

## Patentansprüche

1. Anzündsatz, **dadurch gekennzeichnet, dass** er Calcium-Styphnat, basisches Calciumstyphnat und/oder basisches Kalium-Calcium-Styphnat enthält.

2. Anzündsatz nach Anspruch 1, **dadurch gekennzeichnet, dass** er zusätzlich ein oder mehrere Zusatzstoffe enthält, ausgewählt aus Oxidationsmitteln, Reduktionsmitteln, Sensibilisatoren, Bindemitteln, energiereichen Zuschlägen, Abbrandmoderatoren, Verarbeitungshilfen und/oder Mischungen aus diesen Komponenten.

3. Anzündsatz nach Anspruch 1 oder 2, **dadurch gekennzeichnet, dass** das Oxidationsmittel ausgewählt ist aus den Nitraten der Alkali- oder Erdalkalimetalle oder des Ammoniums wie Natriumnitrat oder Kaliumnitrat, Perchlorate der Alkali- oder Erdalkalimetalle oder des Ammoniums, Peroxide der Erdalkalimetalle, Zinks und/oder Mischungen aus diesen Komponenten.

4. Anzündsatz nach mindestens einem der Ansprüche 1 bis 3, **dadurch gekennzeichnet, dass** das Reduktionsmittel ausgewählt ist aus Aluminium, Titan, Titanhydrid, Bor, Borhydrid, Zirkon, Zirkonhydrid, Silicium, Graphit, Aktivkohle, Ruß und/oder Mischungen aus diesen Komponenten.

5. Anzündsatz nach mindestens einem der Ansprüche 1 bis 4, **dadurch gekennzeichnet, dass** der Sensibilisator ausgewählt ist aus Tetrazen, Kaliumdinitrobenzofuroxanat, Diazodinitrophenol und/oder Mischungen aus diesen Kompenten.

6. Anzündsatz nach mindestens einem der Ansprüche 1 bis 5, **dadurch gekennzeichnet, dass** das Bindemittel ausgewählt ist aus Adhesin, Cellulose sowie deren Derivate, Polyvinylbutyraten, Polynitropolyphenylen, Polynitrophenylether, Plexigum, Polyvinylacetat, Copolymeren und/oder Mischungen aus diesen Komponenten.

7. Anzündsatz nach mindestens einem der Ansprüche 1 bis 6, **dadurch gekennzeichnet, dass** die energiereichen Zuschläge ausgewählt sind Hexogen, Oktogen, Nitropenta und Nitrocellulose und/oder Mischungen aus diesen Komponenten.

8. Anzündsatz nach mindestens einem der Ansprüche 1 bis 7, **dadurch gekennzeichnet, dass** die Abbrandmoderatoren und Verarbeitungshilfen ausgewählt sind aus Nitrocellulose-Kugelpulver, Acetonylacetate, Salicylate, Silikate, Kieselgele, Bornitrid und/oder Mischungen aus diesen Komponenten.

9. Anzündsatz nach mindestens einem der Ansprüche 1 bis 8, **dadurch gekennzeichnet, dass** er 10 bis 90, bevorzugt 20 bis 80, besonders bevorzugt 30 bis 70 Gew.-% Kalium-Calcium-Styphnat, basisches Calciumstyphnat und/oder basisches Kalium-Calcium-Styphnat enthält.

10. Anzündsatz nach mindestens einem der Ansprüche 1 bis 9, **dadurch gekennzeichnet, dass** er 70 bis 99,99, bevorzugt 90 bis 99,9, besonders bevorzugt 90 bis 99 Gew.-% Kalium-Calcium-Styphnat, basisches Calciumstyphnat und/oder basisches Kalium-Calcium-Styphnat enthält.

11. Anzündsatz nach mindestens einem der Ansprüche 1 bis 10, **dadurch gekennzeichnet, dass** er 90 bis 99,99, bevorzugt 95 bis 99 Gew.-% Kalium-Calcium-Styphnat, vorzugsweise basisches Kalium-Calcium-Styphnat, und 0,01 bis 10, bevorzugt 1 bis 5 Gew.-% Bindemittel, vorzugsweise Adhesin, enthält.

12. Verwendung eines Anzündsatzes nach mindestens einem der Ansprüche 1 bis 11 in Anzündsystemen für Fahrzeugsicherheitssystemen, bevorzugt in solchen, die im Motorraum eines Kfz eingesetzt werden, in Anzündsystemen für Munition und/oder Treibkartuschen für Bolzensetzgeräte.

## Claims

1. A primer composition, **characterised in that** it comprises potassium-calcium styphnate, basic calcium styphnate and/or basic potassium-calcium styphnate.

2. A primer composition according to claim 1, **characterised in that** it additionally comprises one or more additives selected from oxidising agents, reducing agents, sensitisers, binders, high-energy additives, combustion moderators, processing aids and/or mixtures of these components.

3. A primer composition according to claim 1 or 2, **characterised in that** the oxidising agent is selected from the nitrates of the alkali or alkaline-earth metals or of ammonium, such as sodium nitrate or potassium nitrate, perchlorates of the alkali or alkaline-earth metals or of ammonium, peroxides of the alkaline-earth metals, of zinc and/or mixtures of these components.

4. A primer composition according to at least one of claims 1 to 3, **characterised in that** the reducing agent is selected from aluminium, titanium, titanium hydride, boron, boron hydride, zirconium, zirconium hydride, silicon, graphite, active carbon, carbon black and/or mixtures of these components.

5. A primer composition according to at least one of claims 1 to 4, **characterised in that** the sensitiser is selected from tetrazene, potassium dinitrobenzofuroxanate, diazodinitrophenol and/or mixtures of these components.

6. A primer composition according to at least one of claims 1 to 5, **characterised in that** the binder is selected from adhesin, cellulose and derivatives thereof, polyvinylbutyrals, polynitropolyphenylene, polynitrophenyl ether, plexigum, polyvinyl acetate, copolymers and/or mixtures of these components.

7. A primer composition according to at least one of claims 1 to 6, **characterised in that** the high-energy additives are selected from hexogen, octogen, nitropenta and nitrocellulose and/or mixtures of these components.

8. A primer composition according to at least one of claims 1 to 7, **characterised in that** the combustion moderators and processing aids are selected from nitrocellulose spherical powders, acetonyl acetates, salicylates, silicates, silica gels, boron nitride and/or mixtures of these components.

9. A primer composition according to at least one of claims 1 to 8, **characterised in that** it comprises from 10 to 90 wt.%, preferably from 20 to 80 wt.%, particularly preferably from 30 to 70 wt.%, potassium-calcium styphnate, basic calcium styphnate and/or basic potassium-calcium styphnate.

10. A primer composition according to at least one of claims 1 to 9, **characterised in that** it comprises from 70 to 99.99 wt.%, preferably from 90 to 99.9 wt.%, particularly preferably from 90 to 99 wt.%, potassium-calcium styphnate, basic calcium styphnate and/or basic potassium-calcium styphnate.

11. A primer composition according to at least one of claims 1 to 10, **characterised in that** it comprises from 90 to 99.99 wt.%, preferably from 95 to 99 wt.%, potassium-calcium styphnate, preferably basic potassium-calcium styphnate, and from 0.01 to 10 wt.%, preferably from 1 to 5 wt.%, binder, preferably adhesin.

12. Use of a primer composition according to at least one of claims 1 to 11 in primer systems for vehicle safety systems, preferably in those which are used in the engine compartment of a motor vehicle, in primer systems for ammunition and/or propellant cartridges for bolt-firing tools.

## Revendications

1. Amorce, **caractérisée en ce qu'**elle contient du styphnate de potassium et de calcium, du styphnate basique de calcium et/ou du styphnate basique de potassium et de calcium.

2. Amorce conforme à la revendication 1, **caractérisée en ce qu'**elle contient en outre un ou plusieurs adjuvant(s) choisi(s) parmi les oxydants, les réducteurs, les sensibilisateurs, les liants, les additifs énergétiques, les modérateurs de combustion, les auxiliaires de traitement et/ou les mélanges de tels composants.

3. Amorce conforme à la revendication 1 ou 2, **caractérisée en ce que** l'oxydant est choisi parmi les nitrates de métal alcalin, de métal alcalino-terreux ou d'ammonium, comme le nitrate de sodium ou le nitrate de potassium, les perchlorates de métal alcalin, de métal alcalino-terreux ou d'ammonium, les peroxydes des métaux alcalino-terreux ou de zinc, et/ou les mélanges de tels composants.

4. Amorce conforme à l'une au moins des revendications 1 à 3, **caractérisée en ce que** le réducteur est choisi parmi de l'aluminium, du titane, de l'hydrure de titane, du bore, les hydrures de bore, du zirconium, de l'hydrure de zirconium, du silicium, du graphite, du charbon actif, du noir de fumée, et/ou les mélanges de tels composants.

5. Amorce conforme à l'une au moins des revendications 1 à 4, **caractérisée en ce que** le sensibilisateur est choisi parmi le tétrazène, le dinitro-benzofuroxanate de potassium, le diazo-dinitro-phénol et/ou les mélanges de ces composants.

6. Amorce conforme à l'une au moins des revendications 1 à 5, **caractérisée en ce que** le liant est choisi parmi une adhésine, la cellulose et ses dérivés, le poly(butyral de vinyle), les polynitro-polyphénylènes, les polynitro-phényl-éthers, les plexigums, le poly(acétate de vinyle), les copolymères et/ou les mélanges de ces composants.

7. Amorce conforme à l'une au moins des revendications 1 à 6, **caractérisée en ce que** les additifs énergétiques sont choisis parmi de l'hexogène, de l'octogène, du tétranitrate de pentaérythritol et de la nitrocellulose et/ou les mélanges de ces composants.

8. Amorce conforme à l'une au moins des revendications 1 à 7, **caractérisée en ce que** les modérateurs de combustion et auxiliaires de traitement sont choisis parmi de la poudre à balles de nitrocellulose, de l'acétate d'acétonyle, les salicylates, les silicates, les gels de silice, le nitrure de bore et/ou les mélanges de ces composants.

9. Amorce conforme à l'une au moins des revendications 1 à 8, **caractérisée en ce qu'**elle contient de 10 à 90 %, de préférence de 20 à 80 % et surtout de 30 à 70 % en poids de styphnate de potassium et de calcium, de styphnate basique de calcium et/ou de styphnate basique de potassium et de calcium.

10. Amorce conforme à l'une au moins des revendications 1 à 9, **caractérisée en ce qu'**elle contient de 70 à 99,99 %, de préférence de 90 à 99,9 % et surtout de 90 à 99 % en poids de styphnate de potassium et de calcium, de styphnate basique de calcium et/ou de styphnate basique de potassium et de calcium.

11. Amorce conforme à l'une au moins des revendications 1 à 10, **caractérisée en ce qu'**elle contient de 90 à 99,99 %, de préférence de 95 à 99 % de styphnate de potassium et de calcium, de préférence du styphnate basique de potassium et de calcium, et de 0,01 à 10 %, de préférence de 1 à 5 %, en poids d'un liant, et de préférence, d'adhésine.

12. Utilisation d'une amorce conforme à l'une au moins des revendications 1 à 11 dans des dispositifs d'amorçage pour systèmes de sécurité de véhicule, de préférence dans de tels systèmes qui sont utilisés dans l'espace moteur d'un véhicule automobile, dans des dispositifs d'amorçage pour munitions et/ou dans des cartouches propulsives pour pistolets plante-goujons.
